# EUROPEAN PATENT SPECIFICATION

(11) **EP 1 937 306 B1**
(45) Date of publication and mention of the grant of the patent: **17.02.2016**
(21) Application number: 06801923.1
(22) Date of filing: 18.08.2006
(51) Int. Cl.: A61K 39/395, C12Q 1/37, C07K 16/00

(54) **PROTEOLYSIS RESISTANT ANTIBODY PREPARATIONS**
PROTEOLYSE-RESISTENTE ANTIKÖRPERZUBEREITUNGEN
PRÉPARATIONS D'ANTICORPS RÉSISTANTS À LA PROTÉOLYSE

(30) Priority: 19.08.2005 US 709712 P; 21.06.2006 US 805396 P
(43) Date of publication of application: 02.07.2008
(73) Proprietor: Janssen Biotech, Inc., Horsham, PA 19044 (US)
(72) Inventor: RAJU, T. Shantha, West Chester, PA 19382 (US); SCALLON, Bernard, Wayne, PA 19087 (US)
(74) Representative: Marshall, Cameron John
(86) International application number: PCT/US2006/032458
(87) International publication number: WO 2007/024743

(56) References cited:
- WO-A1-99/22764
- US-A1- 2003 157 108
- WILSON D S ET AL: "Improved method for pepsinolysis of mouse IgG1 molecules to F(ab')2 fragments", JOURNAL OF IMMUNOLOGICAL METHODS, ELSEVIER SCIENCE PUBLISHERS B.V.,AMSTERDAM, NL, vol. 260, no. 1-2, 1 February 2002 (2002-02-01), pages 29-36, XP004332557, ISSN: 0022-1759, DOI: 10.1016/S0022-1759(01)00514-2
- BENNETT K L ET AL: "Monitoring papain digestion of a monoclonal antibody by electrospray ionization mass spectrometry", ANALYTICAL BIOCHEMISTRY, ACADEMIC PRESS INC, NEW YORK, vol. 245, no. 1, 1 January 1997 (1997-01-01), pages 17-27, XP002297987, ISSN: 0003-2697, DOI: 10.1006/ABIO.1996.9904
- AXFORD ET AL: "Glycosylation and rheumatic disease", BIOCHIMICA ET BIOPHYSICA ACTA. MOLECULAR BASIS OF DISEASE, AMSTERDAM, NL, vol. 1455, no. 2-3, 8 October 1999 (1999-10-08), pages 219-229, XP027585655, ISSN: 0925-4439 [retrieved on 1999-10-08]
- RAJU T S ET AL: "Glycosylation in the Fc domain of IgG increases resistance to proteolytic cleavage by papain", BIOCHEMICAL AND BIOPHYSICAL RESEARCH COMMUNICATIONS, ACADEMIC PRESS INC. ORLANDO, FL, US, vol. 341, no. 3, 17 March 2006 (2006-03-17), pages 797-803, XP024923854, ISSN: 0006-291X, DOI: 10.1016/J.BBRC.2006.01.030 [retrieved on 2006-03-17]

### SEQUENCE LISTING

<110> CENTOCOR, INC.
<120> PROTEOLYSIS RESISTANT ANTIBODY PREPARATIONS
<130> CEN5107PCT
<140> TO BE ASSIGNED
   <141> 2006-08-18
<150> 60/709,712 <151> 2005-08-19
<150> 60/805,396 <151> 2006-06-21
<160> 7
<170> FastSEQ for Windows Version 4.0
<210> 1
   <211> 23
   <212> PRT
   <213> Homo sapiens
<400> 1
   Ala Glu Pro Lys Ser Cys Asp Lys Thr His Thr Cys Pro Pro Cys Pro
    1               5                  10                  15
   Ala Pro Glu Leu Leu Gly Gly
               20
<210> 2
   <211> 17
   <212> PRT
   <213> Homo sapiens
<400> 2
   Cys Pro Pro Leu Lys Glu Cys Pro Pro Cys Pro Ala Pro Pro Val Ala
   1 5 10 15
   Gly
<210> 3
   <211> 18
   <212> PRT
   <213> Homo sapiens
<400> 3
   Cys Asp Thr Pro Pro Pro Cys Pro Arg Pro Cys Pro Ala Pro Glu Leu
   1 5 10 15
   Leu Gly
<210> 4
   <211> 13
   <212> PRT
   <213> Homo sapiens
<400> 4
   Ser Lys Tyr Gly Pro Pro Cys Pro Ser Cys Pro Ala Pro
   1 5 10
<210> 5
   <211> 12
   <212> PRT
   <213> Homo sapiens
<400> 5
   Pro Ala Pro Glu Leu Leu Gly Gly Pro Ser Val Phe
   1 5 10
<210> 6
   <211> 7
   <212> PRT
   <213> Homo sapiens
<400> 6
   Pro Ala Pro Glu Leu Leu Gly
   1 5
<210> 7
   <211> 7
   <212> PRT
   <213> Homo sapiens
<400> 7
   Pro Ala Pro Glu Leu Leu Gly
    1               5

## Description

### Field of the Invention

The invention relates to evaluating the glycoform content of antibodies and, more particularly, to methods of preparing and using antibody preparations that are substantially homogeneous glycoform, for example, unsialylated glycoforms.

### Background

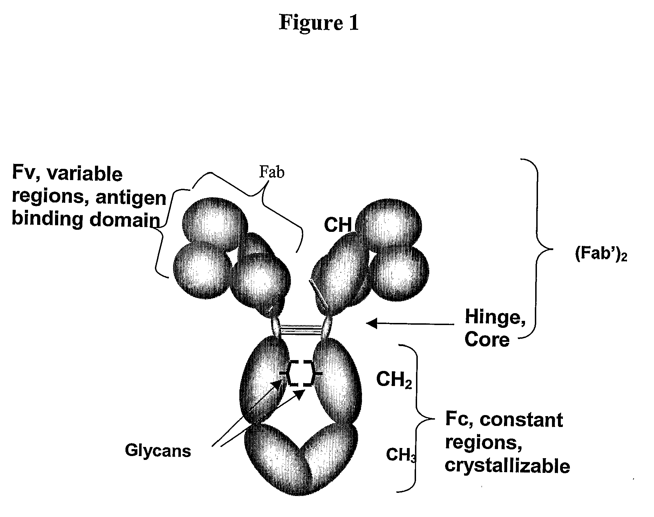

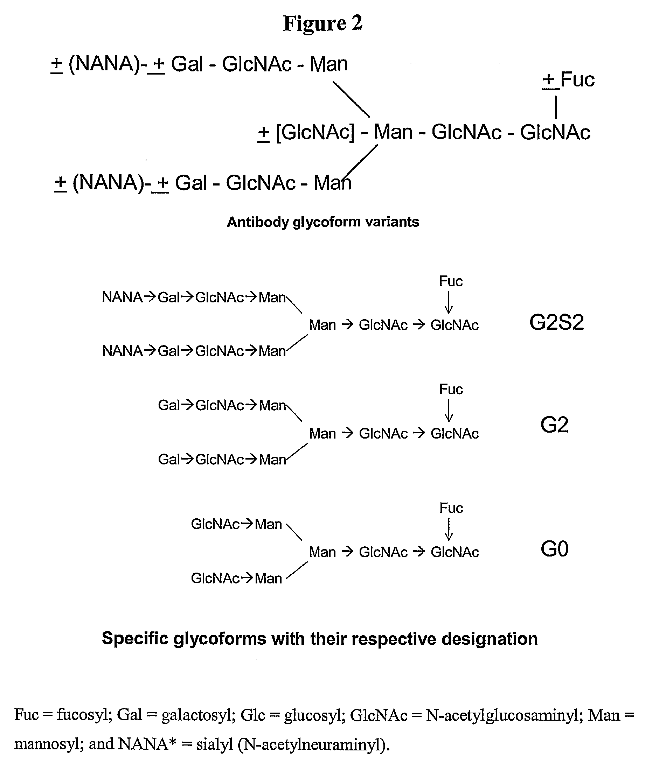

Antibodies are soluble serum glycoproteins that play a significant role in innate immunity. The carbohydrate structures of all naturally produced antibodies at conserved positions in the heavy chain constant regions vary with isotype (Fig. 1). Each isotype possesses a distinct array of N-linked oligosaccharide structures, which variably affect protein assembly, secretion or functional activity (Wright, A., and Morrison, S. L., Trends Biotech. 15:26-32 (1997)). The structure of the attached N-linked oligosaccharides (Fig. 2) varies considerably, depending on the degree of processing, and can include high-mannose, as well as complex biantennary oligosaccharides with or without bisecting GlcNAc and core Fucose residues (Wright, A., and Morrison, S. L., supra). Typically, there is heterogeneous processing of the core oligosaccharide structures attached at a particular glycosylation site such that even monoclonal antibodies exist as multiple glycoforms. Likewise, it has been shown that major differences in antibody glycosylation occur between antibody-producing cell lines, and even minor differences are seen for a given cell line grown under different culture conditions.

Among antibody isotypes (e.g., IgE, IgD, IgA, IgM, and IgG), IgGs are the most abundant with the IgG 1 subclasses exhibiting the most significant degree and array of effector functions. IgG1-type antibodies are the most commonly used antibodies in cancer immunotherapy where ADCC and CDC activity are often deemed important. Structurally, the IgG hinge region and CH2 domains play a major role in the antibody effector functions. The N-linked oligosaccharides present in the Fc region (formed by the dimerization of the hinge, CH2 and CH3 domains) affect the effector functions. These covalently bound oligosaccharides are complex biantennary type structures and are highly heterogeneous (see Fig. 2); NANA, 5-N-acetylneuraminic acid, (NeuAc) or NGNA, 5-N- glycolylneuraminic acid (NeuGc) is typically "sialic acid." Other sialic acids have been found or can be chemically synthesized. A conserved N-linked glycosylation site at Asn297 lies in each CH2 domain. In the mature antibody, the two complex bi-antennary oligosaccharides attached to Asn297 are buried between the CH2 domains, forming extensive contacts with the polypeptide backbone. It has been found that their presence is essential for the antibody to mediate effector functions, such as ADCC (Lifely, M. R., et al., Glycobiology 5:813-822 (1995); Jefferis, R., et al., Immunol Rev. 163:59-76 (1998); Wright, A. and Morrison, S. L., *supra*).

The biological presence and significance of individual saccharides at specific positions has also begun to be explored. For example, the extent of galactosylation of antibodies is affected by age, gender, and disease (Raju, T.S., et al. Glycobiology 2000. 10(5): 477-86). In general, oligosaccharide structures are somewhat species-specific and vary widely. Further, the biological significance of oligosaccharide structures with and without bisecting GlcNAc and core fucose residues has also been studied. Human IgG and many of the recombinantly-produced IgG's contain minor amounts of sialylated (or unsialylated or asialylated) oligosaccharides, however, the vast majority of IgG's contain non-sialylated oligosaccharide structures.

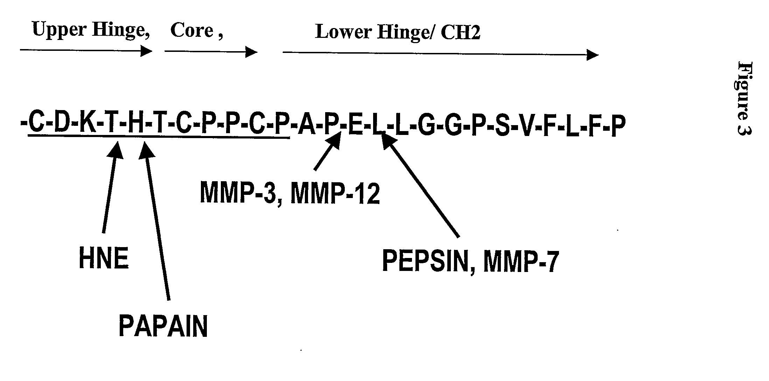

Proteolytic cleavage of antibodies naturally occurs under physiological conditions and can also be an industrial processing step in the production of biologic therapeutics based on antibody structure. Papain-generated or processed therapeutic antibody fragments, Fabs, are gaining more widespread use. Although papain is a plant-derived enzyme, there are a number of protease cleavage sites identified in the IgG1 hinge and they are summarized in Figure 3.

Recombinant IgGs can be converted to IgG fragments, such as Fab and F(ab')2 and Fc, using various proteolytic enzymes (Figs. 1 and 3). The digestion fragments represent a major biotherapeutic classs useful in managing and treating human diseases. Abciximab ((c7E3 Fab, marketed as REOPRO®), is one example of a Fab therapeutic. The 47,615 dalton Fab fragment is purified from cell culture supernatant, digestion with papain and column chromatography. Other examples include: DigiFab (DigiTAB), a preparation of Fab fragments from sheep polyclonal antibodies, for the potential treatment of digoxin poisoning; CroFAb, a preparation of monovalent Fab fragments obtained from sheep immunized with snake venoms, as an antivenom against bites by the four most common North American crotalids (pit vipers) approved in the US in October 2000; and EchiTAb, an antivenom based on Fab fragments of monospecific sheep polyclonal antibodies, for the treatment of bites by the carpet viper (Echis Ocellatus), a snake prevalent in West Africa. Other Fabs in development include: ranibizumab (rhuFab V2; AMD-Fab; Lucentis), a high affinity Fab variant of Genentech's bevacizumab, as a potential treatment for age-related macular degeneration; and 5G1.1, an intravenous humanized monoclonal antibody that prevents the cleavage of human complement component C5 into its pro-inflammatory components, as a potential treatment for several chronic inflammatory diseases, including rheumatoid arthritis (RA), membranous and lupus nephritis, dermatomyositis, and paroxysmal nocturnal hemoglobinuria (PNH).

Other Fab-containing compositions with potential therapeutic use include chemically modified Fabs, such as CDP-870 a humanized anti-TNFalpha-Fab fragment linked to polyethylene glycol (PEG). CDP-870 is derived from a mouse antihuman TNFalpha antibody that was selected for its high-affinity binding and neutralizing potential. Fab fragments of this antibody were constructed by recombinant-DNA technology, humanized and synthesized by fed-batch fermentation in E coli. The yield of this fermentation procedure reached between 300 and 1200 mg protein/l bacterial culture. To enhance plasma half-life, a PEG moiety was added to the Fab fragments. For this purpose, a site-specific conjugation method was developed in which a single cysteine residue was introduced into the hinge region of the Fab fragment for the covalent addition of the hydrophilic polymer (PEG) moiety for the purposes of increasing its circulating half-life. Using a low-cost E coli technology to produce the Fab fragments, allowed the manufacturer (Celltech) to lower the manufacturing costs of CDP-870 by 10- to 20-fold compared with antibodies that are conventionally produced in mammalian cell culture. E. coli do not express glycosylated proteins.

To date, the relationship between glycan presence and composition on the susceptibility of IgGs to proteolytic cleavage from human or other species has not been studied. Therefore, there is a need to understand the relationship between the proteolytic pattern and glycan structure of therapeutically relevant antibody structures for the purposes of efficient antibody production and as a tool for identifying the presence and/or composition of antibody glycans.

### SUMMARY OF THE INVENTION

The invention provides an in vitro method of enhancing the stability of an Fc-containing protein to cleavage by a protease, comprising modifying the sialylation of glycoforms in the Fc region of the Fc-containing protein by treatment with a sialidase, wherein stability to cleavage by papain is enhanced.

The present disclosure comprises a method for enhancing the ability of an antibody preparation to resist cleavage by a protease and methods of using such antibody preparations to treat pathological conditions associated with the presence of elevated levels of proteases, such as cancer. In one embodiment of the method of the invention, the antibody preparation is substantially free of sialylated glycoforms in the Fc region of the antibody. In another aspect of the disclosure, the protease is selected from the group consisting of papain, pepsin, a matrix metalloproteinase including MMP-7, neutrophil elastase (HNE), stromelysin (MMP-3), macrophage elastase (MMP-12), trypsin, chymotrypsin, and other proteases, including glycosylation modification enzymes, e.g., sialidase-A, galactosidase, etc. In one embodiment, the antibody preparation is modified so to be substantially homogeneous with respect to glycoform G0.

In another embodiment, the present disclosure comprises a method for increasing or reducing the ability of an antibody preparation to resist cleavage by a protease and methods of using such antibody preparations in diseases or conditions associated with the presence of increased or reduced levels of proteases.

The present disclosure also comprises a method of enhancing the stability of an antibody with a protease, such as papain, pepsin, a matrix metalloproteinase including MMP-7, neutrophil elastase (HNE), stromelysin (MMP-3), macrophage elastase (MMP-12), trypsin, chymotrypsin, and other proteases, including glycosylation modification enzymes by treating an antibody preparation in vitro with a sialidase and, optionally, further treating the antibody with a β-galactosidase and/or α-galactosidase to remove galactose residues.

The present disclosure further comprises a method for detecting or diagnosing a disease state in a cell or subject, comprising determining the state of glycosylation of antibodies in the cell or subject. The method of determining or diagnosing the disease state may rely on an analysis of the glycoform of the natural or therapeutically administered antibodies in a subject along with a determination of the presence of Fab, F(ab')2, Fv, facb, or Fc fragments in a biological sample from said subject.

### BRIEF DESCRIPTION OF THE SEVERAL VIEWS OF THE DRAWING

- **Fig. 1**: depicts antibody IgG domains showing the relationship between the domains and the major designated cleavage fragments.
- **Fig. 2**: is a schematic depiction of the variations in the biantennary oligosaccharide structure found in human IgG.
- **Fig. 3**: shows the amino acid sequence in the human IgG1 hinge region and cleavage sites for various enzymes.
- **Figs. 4A-G**: are MALDI-TOF-MS recordings with peak information on the identification of species superimposed (+1 is singly charged molecular ion, +2 is doubly charged molecular ion, +3 is triply charged molecular ion and LC is free light chain) and showing the formation of IgG fragments over time during digestion with papain for a glycosylated and deglycosylated preparation: (A) undigested, (B) ½hour, (C) 1 hour, (D) 2 hours, (E) 4 hours, (F) 8 hours, and (G) after 24 hours.
- **Fig. 5**: shows a comparison of the percent peak area of +1 molecular ions of intact IgGs (A) and Fc fragments (B) of glycosylated and deglycosylated IgG samples during papain digestion.
- **Fig. 6**: shows tracings of MALDI-TOF-MS analysis of intact homogeneous IgG glycoform preparations described in Example 5 for G0, G2, and G2S2 glycoforms.
- **Figs. 7A-D**: show tracings of MALDI-TOF-MS analysis of the PGNase released glycans from the various homogeneous glycoform preparations and from the control sample.
- **Fig. 8**: shows tracings of MALDI-TOF-MS analysis of papain digests of homogeneous IgG glycoform preparations G0, G2 and G2S2 along with a control sample subjected to papain digestion at 50:1 ratio at 37°C after 15 minutes with the various peak identities labeled.
- **Fig. 9**: is a graphical representation of the integrated peak area of the intact IgG from MALDI-TOF-MS analysis of papain digests of homogeneous IgG glycoform preparations G0, G2 and G2S2 relative to a control subjected to papain digestion at 50:1 ratio at 37°C at various times.
- **Fig. 10**: is a graphical representation of the integrated peak area of the Fc domain from MALDI-TOF-MS analysis formed during papain digestion of homogeneous IgG glycoform preparations G0, G2 and G2S2 along with a control sample at various times.

### DETAILED DESCRIPTION OF THE INVENTION

### Abbreviations

AA, anthranilic acid; α1,3GT, α-1,3-galactosyltransferase; β1,4GT, β-1,4-galactosyltransferase; α2,3ST, α-2,3-sialyltransferase; ADCC, antibody-dependent cellular cytotoxicity; CDC, complement-dependent cytotoxicity; CMP-Sia, cytidine monophosphate *N*-acetylneuraminic acid; FBS, fetal bovine serum; IgG, immunoglobulin G; MALDI-TOF-MS, matrix-assisted laser/desorption ionization time-of-flight mass spectrometry; NANA, *N*-acetylneuraminic acid isomer of sialic acid; NGNA, *N*-glycolylneuraminic acid isomer of sialic acid; PNGase F, peptide *N-*glycosidase F; HPLC, reversed phase high-performance liquid chromatography; SA, Sinapic acid; Sia, sialic acid; SDHB, dihydroxybenzoic acid containing sodium chloride; UDP-Gal, uridine diphosphate galactose; UDP-GlcNAc, uridine diphosphate *N*-acetylglucosamine.

### Definitions

The terms "Fc," "Fc-containing protein" or "Fc-containing molecule" as used herein refer to a monomeric, dimeric or heterodimeric protein having at least an immunoglobulin CH2 and CH3 domain. The CH2 and CH3 domains can form at least a part of the dimeric region of the protein/molecule (e.g., antibody).

The term "antibody" is intended to encompass antibodies, digestion fragments, specified portions and variants thereof, including, without limitation, antibody mimetics or comprising portions of antibodies that mimic the structure and/or function of an antibody or specified fragment or portion thereof, including, without limitation, single chain antibodies, single domain antibodies, minibodies, and fragments thereof. Functional fragments include antigen-binding fragments that bind to the target antigen of interest. For example, antibody fragments capable of binding to a target antigen or portions thereof, including, but not limited to, Fab (e.g., by papain digestion), Fab' (e.g., by pepsin digestion and partial reduction) and F(ab')₂ (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the term antibody (see, e.g., Colligan, Immunology, supra).

The term "monoclonal antibody" as used herein is a specific form of Fc-containing fusion protein comprising at least one ligand binding domain which retains substantial homology to at least one of a heavy or light chain antibody variable domain of at least one species of animal antibody.

### Enzymatic Digestion of Antibodies

Due to their high affinity target binding, Fabs also provide an ideal targeting moiety, for e.g., conjugation of toxins or to embed in more complex structures, such as liposomes. As an enhancement to long circulating lipid vesicles carrying encapsulated drug, targeted or immunoliposomes are expected to enable more precise delivery of actives to diseased or pathogenic tissue while sparing normal cells thereby reducing side effects. The use of IgG and Fab as targeting moieties for therapeutic liposomes is disclosed in US4957735 and Maruyama et al. (1995) Biochim Biophys Acta 1234: 74-80.

Papain is a sulfhydryl protease that has been used to digest IgG antibodies into either Fab or F(ab')2 fragments, depending on whether L-cysteine is present or absent during the reaction, respectively. Prolonged treatment, or excessive amounts of papain, typically results in overdigestion of the Fc domain, although the Fab domains often remain resistant to overdigestion with papain. This is because the Fc domain contains additional (secondary) papain cleavage sites (Fig. 1). The histidine residue is the C-terminal position of abciximab when papain digestion is performed in the presence of cysteine.

| | |
|---|---|
| Human IgG1: | A-E-P-K-S-C-D-K-T-H-T-C-P-P-C-P-A-P-E-L-L-G-G |
| Human IgG2: | C-P-P -L-K-E-C-P-P-C-P-A-P-P-_-V-A-G |
| Human IgG3: | C-D-T-P-P-P-C-P-R-P-C-P-A-P-E-L-L-G |
| Human IgG4: | S-K-Y-G-P-P- C-P-S-C-P-A-P |

While papain is an industrially useful enzyme, it is of plant origin originally isolated from the green fruit and leaves of *Carica papaya* (*Caricaceae* spp). An industrially useful mammalian enzyme, is pepsin. Pepsin is autoactivated and active at low pH as it is a normal component of the gastric fluid secreted into the lumen of the stomach after eating. Low levels of the precursor enzyme pepsinogen can be found in the serum but, since activation and activity are acid dependent, is not physiologically relevant to circulating antibodies. Pepsin cleaves human IgG1 between the leucine₂₃₄-leucine₂₃₅ in the lower hinge. This cleavage site is downstream from the hinge core (-C-P-P-C-) containing two cysteine residues that link the two heavy chains via disulfide bonds creating a F(ab')₂ molecule which is bivalent for antigen binding.

The lower hinge/CH2 region, P-A-P-E-L-L-G-G-P-S-V-F is within the domain where cleavage sites exist for MMP-3 and MMP-12 (P-A-P*E-L-L-G for each) as well as pepsin and MMP-7 (P-A-P-E-L*L-G for each). In addition, a group of physiologically relevant enzymes; neutrophil elastase (HNE), stromelysin (MMP-3) and macrophage elastase (MMP-12) cleave IgG at different positions to generate subtly different F(ab')₂, Fab and Fc fragments (Fig. 3).

It was unexpectedly found that the level of glycoslylation of the Fc alters the susceptibility to enzymatic degradation of said antibodies, resulting in modulation of various aspects of the production processes and biological actions of said antibodies. More specifically, during the course of these experiments it was discovered that the Fc of glycosylated Abs is more resistant to papain digestion than that of deglycosylated, aglycosylated or non-glycosylated Abs. Substantially deglycosylated, aglycosylated or non-glycosylated shall mean that most of the actual and/or potential glycosylation sites are unoccupied (with glycan), i.e., are not glycosylated.

The present disclosure further comprises a method for controlling the properties of an Fc-containing molecule by altering the glycosylation of the Fc's CH2 domains and the use of the altered Fc-containing molecules.

The presence or absence of glycan in the Fc-containing molecule affects the affinity for one or more of the FcγRI, FcγRIIA, and FcγRIIIA receptors, ADCC activity, macrophage or monocyte activation, and serum half-life (Lifely et al., Jeffreis, and Wright and Morrison, *supra*). Therefore, since proteolytic degradation is a measure of glycosylation and glycosylation is a requirement for the secondary functions of an IgG-class antibody, susceptibility to proteolysis becomes a marker for the above mentioned functions of said IgG-class antibody. For example, sialic acid has a net negative charge at physiological pH and, thus, the presence of sialic acid in the Fc-bound carbohydrate might be expected to alter the three-dimensional structure and hence conformation of the CH2 domain and thereby affect Fc accessibility by proteolytic enzymes. Accordingly, the sialic acid content of the oligosaccharide attached to the CH2 domain is a determinant of proteolytic susceptibility and proteolytic cleavage rate is a measure of sialic acid content of the IgG or other Fc-containing protein.

### Enrichment of Glycoforms of Fc-containing Proteins

One approach to preparing sublots of a particular Fc-containing protein that differ in glycan content and structure is to take an Fc-containing protein preparation with heterogeneous Fc oligosaccharides, including both glycosylated and aglycosylated molecules, and pass it over a column containing an immobilized lectin that has differential affinity for, for example, sialylated and asialylated oligosaccharides. The nonbinding flow-through (T, through) or the column unbound fraction can be separated from the bound fraction (B, bound), the latter collected while passing elution buffer through the column. It may also be possible to separately collect a weakly bound fraction or the column retarded fraction (R, retarded), for example, by collecting Fc-containing protein that elutes during continued washing of the column with the original sample buffer. Depending on the lectin used, the binding fraction is expected to have a higher saccharide, e.g., sialic acid, content therefore oligosaccharide content, than the non-binding fraction.

Examples of lectins that may enrich for sialylated or asialylated Fc-containing proteins are the lectin from *Maackia amurensis* (MAA), which specifically binds oligosaccharides with terminal sialic acid, and the lectin wheat germ agglutinin (WGA), which specifically binds oligosaccharides with either terminal sialic acid or terminal N-acetylglucosamine (GlcNAc). Another example is the lectin Ricin I (RCA), which binds oligosaccharides with terminal galactose. In the latter example, the non-binding flow-through fraction may be enriched for sialylated Fc-containing molecules. Other lectins known in the art include those provided by Vector labs and EY labs.

### Enzymatic Modification of Fc-containing proteins

An alternative approach for preparing sublots of an Fc-containing protein that differ in glycan content is to treat a portion of an Fc-containing protein preparation with a saccharase, such as a fucosidase or sialidase enzyme, thereby removing specific sugar residues, e.g., fucose or sialic acids. The resulting afucosylated or asialylated material can be compared to the original, partially fucosylated or sialylated material for differences in biological activity.

Addition of saccharides to the Fc region can also be achieved using *in vitro* glycosylation methods. Glycosyltransferases naturally function to synthesize oligosaccharides. They produce specific products with excellent stereochemical and regiochemical geometry. The transfer of glycosyl residues results in the elongation or synthesis of an oligo- or polysaccharide. A number of glycosyltransferase types have been described, including sialyltransferases, fucosyltransferases, galactosyltransferases, mannosyltransferases, N-acetylgalactosaminyltransferases, N-acetylglucosaminyltransferases and the like.

Glycosyltransferases which are useful in the present disclosure include, for example, α-sialyltransferases, α-glucosyltransferases, α-galactosyltransferases, α-fucosyl- transferases, α-mannosyltransferases, α-xylosyltransferases, α-N-acetylhexosaminyltransferases, β-sialyltransferases, β-glucosyltransferases, β-galactosyltransferases, β-fucosyltransferases, β-mannosyltransferases, β-xylosyltransferases, and β-N-acetylhexosaminyltransferases, such as those from Neisseria meningitidis, or other bacterial sources, and those from rat, mouse, rabbit, cow, pig, human and insect and viral sources. Preferably, the glycosyltransferase is a truncation variant of glycosyltransferase enzyme in which the membrane-binding domain has been deleted.

Exemplary galactosyltransferases include α(1,3) galactosyltransferase (E.C. No. 2.4.1.151, see, e.g., Dabkowski et al., Transplant Proc. 25:2921 (1993) and Yamamoto et al. Nature 345:229-233 (1990)) and α(1,4) galactosyltransferase (E.C. No. 2.4.1.38). Other glycosyltransferases can be used, such as a sialyltransferase.

An α(2,3)sialyltransferase, often referred to as the sialyltransferase, can be used in the production of sialyl lactose or higher order structures. This enzyme transfers sialic acid (NeuAc) from CMP-sialic acid to a Gal residue with the formation of an α-linkage between the two saccharides. Bonding (linkage) between the saccharides is between the 2- position ofNeuAc and the 3-position of Gal. An exemplary α(2,3)sialyltransferase referred to as α (2,3)sialyltransferase (EC 2.4.99.6) transfers sialic acid to the non- reducing terminal Gal of a Galβ1→3Glc disaccharide or glycoside. See, Van den Eijnden et al., J. Biol. Chem., 256:3159 (1981), Weinstein et al., J. Biol. Chem., 257:13845 (1982) and Wen et al., J. Biol. Chem., 267:21011 (1992). Another exemplary α-2,3- sialyltransferase (EC 2.4.99.4) transfers sialic acid to the non- reducing terminal Gal of the disaccharide or glycoside. See, Rearick et al., J. Biol. Chem., 254:4444 (1979) and Gillespie et al., J. Biol. Chem., 267:21004 (1992). Further exemplary enzymes include Gal-β-1,4- GlcNAc α-2,6 sialyltransferase (See, Kurosawa et al. Eur. J. Biochem. 219: 375-381 (1994)).

Other glucosyltransferases particularly useful in preparing oligosaccharides of the disclosure are the mannosyltransferases including α(1,2) mannosyltransferase, α(1,3) mannosyltransferase, β(1,4) mannosyltransferase, Dol-P-Man synthase, OCh1, and Pmt1.

Still other glucosyltransferases include N-acetylgalactosaminyltransferases including α(1,3) N-acetylgalactosaminyltransferase, β(1,4) N- acetylgalactosaminyltransferases (Nagata et al. J. Biol. Chem. 267:12082-12089 (1992) and Smith et al. J. Biol Chem. 269:15162 (1994)) and polypeptide N-acetylgalactosaminyltransferase (Homa et al. J. Biol Chem. 268:12609 (1993)). Suitable N-acetylglucosaminyltransferases include GnTI (2.4.1.101, Hull et al., BBRC 176:608 (1991)), GnTII, and GnTIII (Ihara et al. J. Biolchem. 113:692 (1993)), GnTV (Shoreiban et al. J. Biol. Chem. 268: 15381 (1993)).

For those embodiments in which the method is to be practiced on a commercial scale, it can be advantageous to immobilize the glycosyl transferase on a support. This immobilization facilitates the removal of the enzyme from the batch of product and subsequent reuse of the enzyme. Immobilization of glycosyl transferases can be accomplished, for example, by removing from the transferase its membrane-binding domain, and attaching in its place a cellulose-binding domain. One of skill in the art will understand that other methods of immobilization could also be used and are described in the available literature.

Because the acceptor substrates can essentially be any monosaccharide or oligosaccharide having a terminal saccharide residue for which the particular glycosyl transferase exhibits specificity, substrate may be substituted at the position of its non-reducing end. Thus, the glycoside acceptor may be a monosaccharide, an oligosaccharide, a fluorescent-labeled saccharide, or a saccharide derivative, such as an aminoglycoside antibiotic, a ganglioside, or a glycoprotein including antibodies and other Fc-containing proteins. In one group of preferred embodiments, the glycoside acceptor is an oligosaccharide, preferably, Galβ(1-3)GlcNAc, Galβ(1-4)GlcNAc, Galβ(1- 3)GalNAc, Galβ(1-4)GalNAc, Man α(1,3)Man, Man α(1,6)Man, or GalNAcβ(1-4)-mannose. In a particular preferred embodiment, the oligosaccharide acceptor is attached to the CH2 domain of an Fc-containing protein.

The use of activated sugar substrate, i.e., sugar-nucleoside phosphate, can be circumvented by either using a regenerating reaction concurrently with the glycotransferase reaction (also known as a recycling system). For example, as taught in, e.g., U.S. Pat. 6,030,815, a CMP-sialic acid recycling system utilizes CMP-sialic acid synthetase to replenish CMP-sialic acid (CMP-NeuAc) as it reacts with a sialyltransferase acceptor in the presence of a α(2,3)sialyltransferase to form the sialyl-saccharide. The CMP-sialic acid regenerating system useful in the disclosure comprises cytidine monophosphate (CMP), a nucleoside triphosphate (for example, adenosine triphosphate (ATP), a phosphate donor (for example, phosphoenolpyruvate or acetyl phosphate), a kinase (for example, pyruvate kinase or acetate kinase) capable of transferring phosphate from the phosphate donor to nucleoside diphosphates and a nucleoside monophosphate kinase (for example, myokinase) capable of transferring the terminal phosphate from a nucleoside triphosphate to CMP. The α(2,3)sialyltransferase and CMP- sialic acid synthetase can also be viewed as part of the CMP-sialic acid regenerating system as removal of the activated sialic acid serves to maintain the forward rate of synthesis. The synthesis and use of sialic acid compounds in a sialylation procedure using a phagemid comprising a gene for a modified CMP-sialic acid synthetase enzyme is disclosed in international application WO 92/16640, published October 1, 1992.

An alternative method of preparing oligosaccharides is through the use of a glycosyltransferase and activated glycosyl derivatives as donor sugars obviating the need for sugar nucleotides as donor sugars as taught in U.S. Pat. 5,952,203. The activated glycosyl derivatives act as alternates to the naturally-occurring substrates, which are expensive sugar-nucleotides, usually nucleotide diphosphosugars or nucleotide monophosphosugars in which the nucleotide phosphate is α-linked to the 1-position of the sugar.

Activated glycoside derivatives which are useful include an activated leaving group, such as, for example, fluoro, chloro, bromo, tosylate ester, mesylate ester, triflate ester and the like. Preferred embodiments of activated glycoside derivatives include glycosyl fluorides and glycosyl mesylates, with glycosyl fluorides being particularly preferred. Among the glycosyl fluorides, α-galactosyl fluoride, α-mannosyl fluoride, α-glucosyl fluoride, α- fucosyl fluoride, α-xylosyl fluoride, α-sialyl fluoride, alpha-N-acetylglucosaminyl fluoride, α-N-acetylgalactosaminyl fluoride, β-galactosyl fluoride, β-mannosyl fluoride, β-glucosyl fluoride, β-fucosyl fluoride, β-xylosyl fluoride, beta-sialyl fluoride, β-N-acetylglucosaminyl fluoride and β-N-acetylgalactosaminyl fluoride are most preferred.

Glycosyl fluorides can be prepared from the free sugar by first acetylating the sugar and then treating it with HF/pyridine. Acetylated glycosyl fluorides may be deprotected by reaction with mild (catalytic) base in methanol (e.g., NaOMe/MeOH). In addition, many glycosyl fluorides are commercially available. Other activated glycosyl derivatives can be prepared using conventional methods known to those of skill in the art. For example, glycosyl mesylates can be prepared by treatment of the fully benzylated hemiacetal form of the sugar with mesyl chloride, followed by catalytic hydrogenation to remove the benzyl groups.

A further component of the reaction is a catalytic amount of a nucleoside phosphate or analog thereof. Nucleoside monophosphates which are suitable for use in the present disclosure include, for example, adenosine monophosphate (AMP), cytidine monophosphate (CMP), uridine monophosphate (UMP), guanosine monophosphate (GMP), inosine monophosphate (IMP) and thymidine monophosphate (TMP). Nucleoside triphosphates suitable for use in accordance with the present disclosure include adenosine triphosphate (ATP), cytidine triphosphate (CTP), uridine triphosphate (UTP), guanosine triphosphate (GTP), inosine triphosphate (ITP) and thymidine triphosphate (TTP). A preferred nucleoside triphosphate is UTP. Preferably, the nucleoside phosphate is a nucleoside diphosphate, for example, adenosine diphosphate (ADP), cytidine diphosphate (CDP), uridine diphosphate (UDP), guanosine diphosphate (GDP), inosine diphosphate (IDP) and thymidine diphosphate (TDP). A preferred nucleoside diphosphate is UDP. As noted above, the present disclosure can also be practiced with an analog of the nucleoside phosphates. Suitable analogs include, for example, nucleoside sulfates and sulfonates. Still other analogs include simple phosphates, for example, pyrophosphate.

One procedure for modifying recombinant proteins produced, in e.g., murine cells wherein the hydroxylated form of sialic acid predominates (NGNA), is to treat the protein with sialidase, to remove NGNA-type sialic acid, followed by enzymatic galactosylation using the reagent UDP-Gal and betal,4 Galtransferase to produce highly homogeneous G2 glycoforms. The preparation can then, optionally, be treated with the reagent CMP-NANA and alpha-2,3 sialyltransferase to give highly homogeneous G2S2 glycoforms.

For purposes of this invention, substantially homogeneous for a glycoform shall mean about 85% or greater of that glycoform and, preferably about 95% or greater of that glycoform.

### Structural Characterization of Sialic Acid Variants

For structural characterization of sialic acid variants containing oligosaccharides, the glycoprotein preparations including antibody preparations were treated with peptide-N-glycosidase F to release the N-linked oligosaccharides. The enzyme peptide-N-glycosidase F (PNGase F) cleaves asparagines-linked oligosaccharides. The released oligosaccharides are fluorescently labeled with anthranilic acid (2-aminobenzoic acid), purified and analyzed by HPLC as described (see Anumula, K. R. and Dhume ST. Glycobiology. 1998 Jul;8(7):685-94). Alternatively, the oligosaccharides released can be subjected to MALDI-TOF-MS, as described herein or to EsI-MS. The oligosaccharides separated as various discreet molecular weights, such as G0, G1, G2, G2S1 and G2S2, by these methods can be detected and quantified.

### Biological Characterization of Glycoform Variants

Fc-containing proteins can be compared for functionality by several well-known in vitro assays. In particular, affinity for members of the FcγRI, FcγRII, and FcγRIII family of Fcγ receptors is of interest. These measurements could be made using recombinant soluble forms of the receptors or cell-associated forms of the receptors. In addition, affinity for FcRn, the receptor responsible for the prolonged circulating half-life of IgGs, can be measured, for example, by BIAcore using recombinant soluble FcRn. Cell-based functional assays, such as ADCC assays and CDC assays, provide insights into the likely functional consequences of particular variant structures. In one embodiment, the ADCC assay is configured to have NK cells be the primary effector cell, thereby reflecting the functional effects on the FcγRIIIA receptor. Phagocytosis assays may also be used to compare immune effector functions of different variants, as can assays that measure cellular responses, such as superoxide or inflammatory mediator release. In vivo models can be used as well, as, for example, in the case of using variants of anti-CD3 antibodies to measure T cell activation in mice, an activity that is dependent on Fc domains engaging specific ligands, such as Fcγ receptors.

### Protein Production Processes

Different processes involved with the production of Fc-containing proteins can impact Fc oligosaccharide structure. In one instance, the host cells secreting the Fc-containing protein are cultured in the presence of serum, e.g., fetal bovine serum (FBS) that was not previously subjected to an elevated heat treatment (for example, 56°C for 30 minutes). This can result in Fc-containing protein that contains no, or very low amounts of, sialic acid, due to the natural presence in the serum of active sialidase enzymes that can remove sialic acid from the Fc-containing proteins secreted from those cells. In another embodiment, the cells secreting the Fc-containing protein are cultured either in the presence of serum that was subjected to an elevated heat treatment, thereby inactivating sialidase enzymes, or in the absence of serum or other medium components that may contain sialidase enzymes, such that the Fc-containing protein has higher or lower levels of glycosylation or glycosylation variants.

In another embodiment, the conditions used to purify and further process Fc-containing proteins are established that will favor optimal glycan content. In one embodiment, the conditions produce maximal or minimal oligosaccharide content or cause the transformation of the expressed Fc-containing polypeptide in a predominant glycoform. For example, because sialic acid is acid-labile, prolonged exposure to a low pH environment, such as following elution from protein A chromatography column or viral inactivation efforts, may lead to a reduction in sialic acid content.

### Host Cell Selection or Host Cell Engineering

As described herein, the host cell chosen for expression of the recombinant Fc-containing protein or monoclonal antibody is an important contributor to the final composition, including, without limitation, the variation in composition of the oligosaccharide moieties decorating the protein in the immunoglobulin CH2 domain. Thus, one aspect of the invention involves the selection of appropriate host cells for use and/or development of a production cell expressing the desired therapeutic protein.

In one embodiment in which the sialic acid content is controlled, the host cell is a cell that is naturally deficient or devoid of sialyltransferases. In another embodiment, the host cell is genetically modified to be devoid of sialyltransferases. In a further embodiment, the host cell is a derivative host cell line selected to express reduced or undetectable levels of sialyltransferases. In yet another embodiment, the host cell is naturally devoid of, or is genetically modified to be devoid of, CMP-sialic acid synthetase, the enzyme that catalyzes the formation of CMP-sialic acid, which is the source of sialic acid used by sialyltransferase to transfer sialic acid to the antibody. In a related embodiment, the host cell maybe naturally devoid of, or is genetically modified to be devoid of, pyruvic acid synthetase, the enzyme that forms sialic acid from pyruvic acid.

In an additional embodiment, the host cell may be naturally devoid of, or is genetically modified to be devoid of, galactosyltransferases, such that antibodies expressed in said cells lack galactose. Without galactose, sialic acid will not be attached. In a separate embodiment, the host cell cell may naturally overexpress, or be genetically modified to overexpress, a sialidase enzyme that removes sialic acid from antibodies during production. Such a sialidase enzyme may act intracellularly on antibodies before the antibodies are secreted or be secreted into the culture medium and act on antibodies that have already been secreted into the medium and may further contain a galactase. Methods of selecting cell lines with altered glycosylases and which express glycoproteins with altered carbohydrate compositions have been described (Ripka and Stanley, 1986. Somatic Cell Mol Gen 12:51-62; US2004/0132140). Methods of engineering host cells to produce antibodies with altered glycosylation patterns resulting in enhanced ADCC have been taught in, e.g., U.S. Pat. 6,602,864, wherein the host cells harbor a nucleic acid encoding at least one glycoprotein modifying glycosyl transferase, specifically β (1,4)-N-acetylglucosaminyltranferase III (GnTIII).

Other approaches to genetically engineering the glycosylation properties of a host cell through manipulation of the host cell glycosyltransferase involve eliminating or suppressing the activity, as taught in EP1,176,195, specifically, alpha1,6 fucosyltransferase (FUT8 gene product). It would be known to one skilled in the art to practice the methods of host cell engineering in other than the specific examples cited above. Further, the engineered host cell may be of mammalian origin or may be selected from COS-1, COS-7, HEK293, BHK21, CHO, BSC-1, Hep G2, 653, SP2/0, 293, HeLa, myeloma, lymphoma, yeast, insect or plant cells, or any derivative, immortalized or transformed cell thereof.

In another embodiment, the method of suppressing or eliminating the activity of the enzyme required for oligosaccharide attachment may be selected from the group consisting of gene silencing, such as by the use of siRNA, genetic knock-out, or addition of an enzyme inhibitor, such as by co-expression of an intracellular Ab or peptide specific for the enzyme that binds and blocks its enzymatic activity, and other known genetic engineering techniques. In another embodiment, a method of enhancing the expression or activity of an enzyme that blocks saccharide attachment, or a saccharidase enzyme that removes sugars that are already attached, may be selected from the group consisting of: transfections with recombinant enzyme genes, transfections of transcription factors that enhance enzyme RNA synthesis, and genetic modifications that enhance stability of enzyme RNA, all leading to enhanced activity of enzymes, such as sialidases, that result in lower levels of sialic acid in the purified product. In another embodiment, specific enzyme inhibitors may be added to the cell culture medium. Alternatively, the host cell may be selected from a species or organism incapable of glycosylating polypeptides, e.g. a prokaryotic cell or organism, such as and of the natural or engineered *E. coli spp*, *Klebsiella spp*., or *Pseudomonas spp.*

### Antibodies

An antibody described in this application can include or be derived from any mammal, such as, but not limited to, a human, a mouse, a rabbit, a rat, a rodent, a primate, or any combination thereof and includes isolated human, primate, rodent, mammalian, chimeric, humanized and/or CDR-grafted antibodies, immunoglobulins, cleavage products and other specified portions and variants thereof. The invention also relates to antibody encoding or complementary nucleic acids, vectors, host cells, compositions, formulations, devices, transgenic animals, transgenic plants, and methods of making and using thereof, as described herein together as combined with what is known in the art.

The antibodies or Fc-fusion proteins described herein can be derived in several ways well known in the art. In one aspect, the antibodies are conveniently obtained from hybridomas prepared by immunizing a mouse with the target peptides. The antibodies can thus be obtained using any of the hybridoma techniques well known in the art, see, e.g., Ausubel, et al., ed., Current Protocols in Molecular Biology, John Wiley & Sons, Inc., NY, NY (1987-2001); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2001); Colligan et al., Current Protocols in Protein Science, John Wiley & Sons, NY, NY, (1997-2001).

The antibodies or Fc-fusion proteins or components and domains thereof may also be obtained from selecting from libraries of such domains or components, e.g., a phage library. A phage library can be created by inserting a library of random oligonucleotides or a library of polynucleotides containing sequences of interest, such as from the B-cells of an immunized animal or human (Smith, G.P. 1985. Science 228: 1315-1317). Antibody phage libraries contain heavy (H) and light (L) chain variable region pairs in one phage allowing the expression of single-chain Fv fragments or Fab fragments (Hoogenboom, et al. 2000, Immunol. Today 21(8) 371-8). The diversity of a phagemid library can be manipulated to increase and/or alter the immunospecificities of the monoclonal antibodies of the library to produce and subsequently identify additional, desirable, human monoclonal antibodies. For example, the heavy (H) chain and light (L) chain immunoglobulin molecule encoding genes can be randomly mixed (shuffled) to create new HL pairs in an assembled immunoglobulin molecule.

Additionally, either or both the H and L chain encoding genes can be mutagenized in a complementarity determining region (CDR) of the variable region of the immunoglobulin polypeptide, and subsequently screened for desirable affinity and neutralization capabilities. Antibody libraries also can be created synthetically by selecting one or more human framework sequences and introducing collections of CDR cassettes derived from human antibody repertoires or through designed variation (Kretzschmar and von Ruden 2000, Current Opinion in Biotechnology, 13:598-602). The positions of diversity are not limited to CDRs, but can also include the framework segments of the variable regions or may include other than antibody variable regions, such as peptides.

Other libraries of target binding components which may include other than antibody variable regions are ribosome display, yeast display, and bacterial displays. Ribosome display is a method of translating mRNAs into their cognate proteins while keeping the protein attached to the RNA. The nucleic acid coding sequence is recovered by RT-PCR (Mattheakis, L.C. et al. 1994. Proc. Natl. Acad. Sci. USA 91, 9022). Yeast display is based on the construction of fusion proteins of the membrane-associated alpha-agglutinin yeast adhesion receptor, aga1 and aga2, a part of the mating type system (Broder, et al. 1997. Nature Biotechnology, 15:553-7). Bacterial display is based on fusion of the target to exported bacterial proteins that associate with the cell membrane or cell wall (Chen and Georgiou 2002. Biotechnol Bioeng, 79:496-503).

In comparison to hybridoma technology, phage and other antibody display methods afford the opportunity to manipulate selection against the antigen target in vitro and without the limitation of the possibility of host effects on the antigen or vice versa.

Also described is a method for producing an antibody or Fc-fusion protein, comprising translating the encoding nucleic acid under conditions in vitro, in vivo or in situ, such that the peptide or antibody is expressed in detectable or recoverable amounts.

While having described the invention in general terms, the embodiments of the invention will be further disclosed in the following examples.

### EXAMPLE 1: ISOLATION OF FC DOMAIN FROM IGG

Papain was obtained from Sigma and PNGase F (peptide N-glycosidase F) was obtained from New England Biolabs. Sinapic acid was obtained from Fluka. MALDI-TOF-MS analyses were carried out using Voyager DE Biospectrometry workstation (Applied BioSystems, Foster City, CA).

Antibody samples were deglycosylated by treating with PNGase F in 20 mM Tris-HCl buffer, pH 7.0. The deglycosylated antibody samples were purified on a protein A column (HiTrap Protein A cartridges were obtained from Amersham Biosciences) and analyzed by MALDI-TOF-MS for purity.

Antibody samples (at ∼1 mg/ml, before and after deglycosylation) were treated with papain (1:50, w/w) in 20 mM Tris-HCl buffer, pH 7.0, containing 2 mM L-cysteine and aliquots were withdrawn at fixed time intervals (0, 15, 30, 60, 90 minutes followed by at 2, 3, 4, 5, 6, 8 and 24 hrs). The aliquots (about 2 µl) were immediately mixed with 2 µl of matrix solution (the matrix solution was prepared by dissolving 10 mg sinapic acid in 1.0 ml of 50% acetonitrile in water containing 0.1% trifluoroacetic acid) and 2 µl of this solution was loaded onto the MALDI target plate and allowed to air dry prior to the analysis.

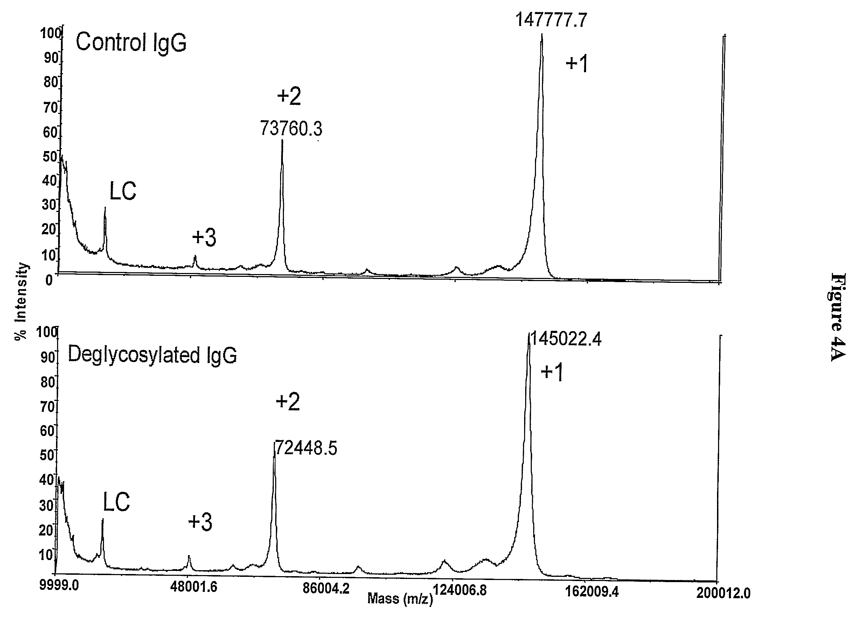

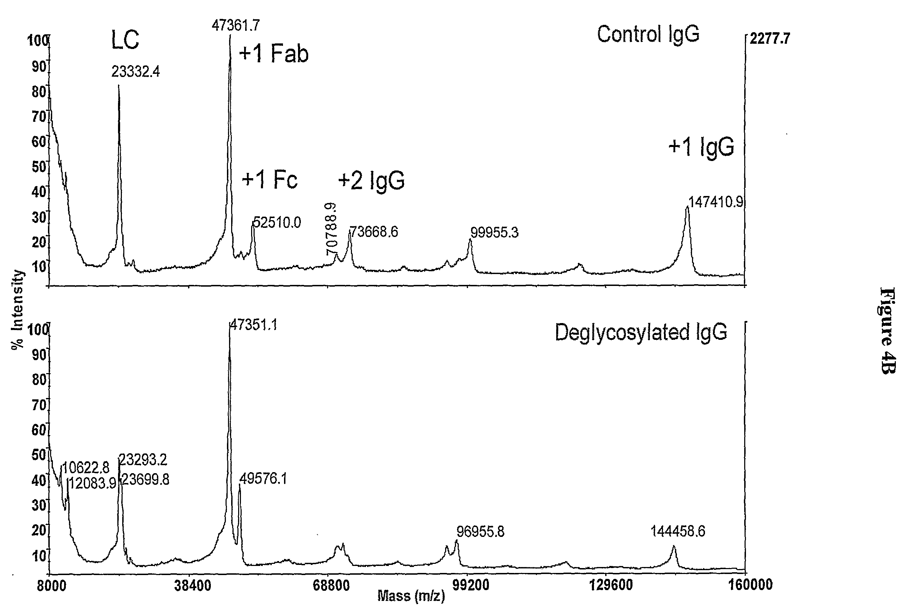

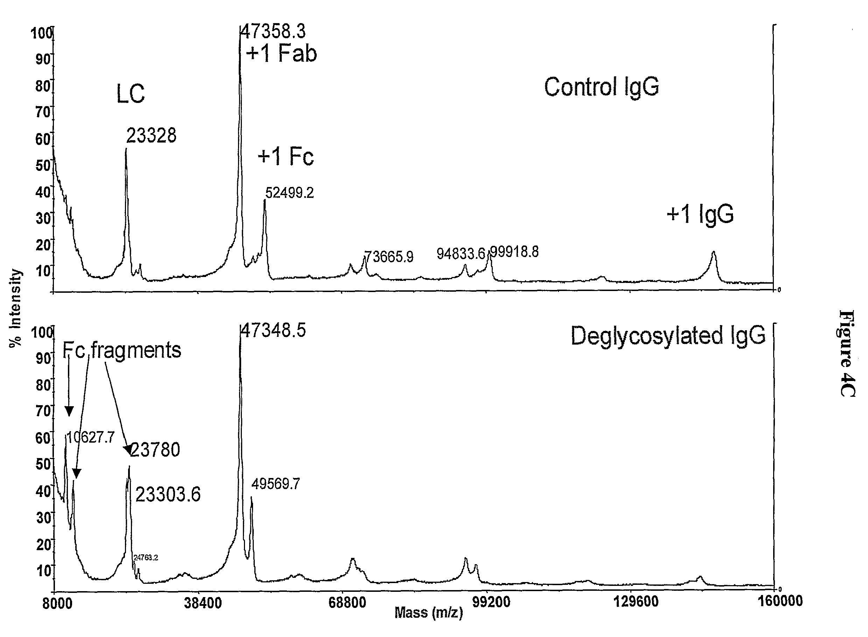

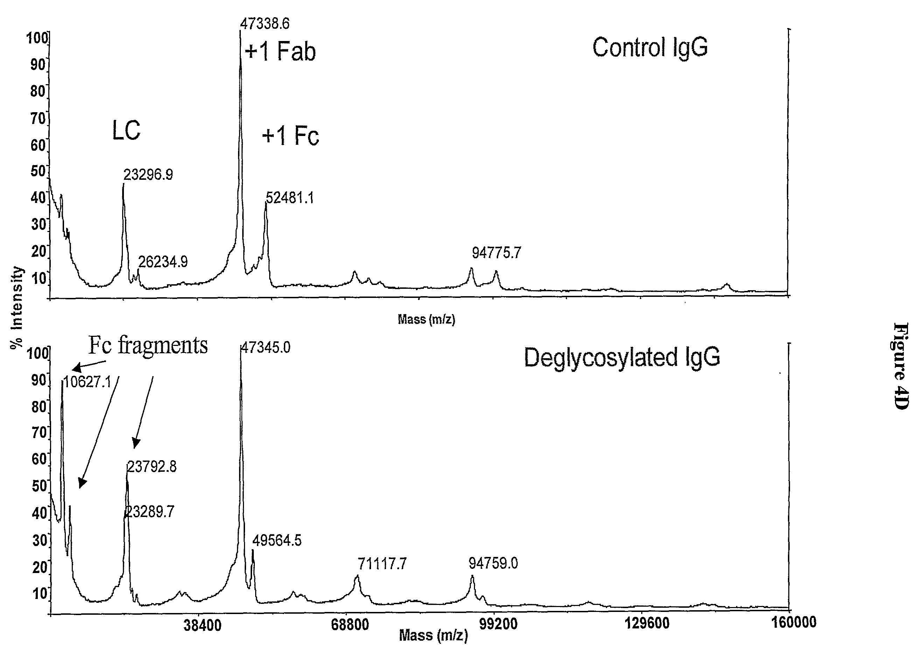

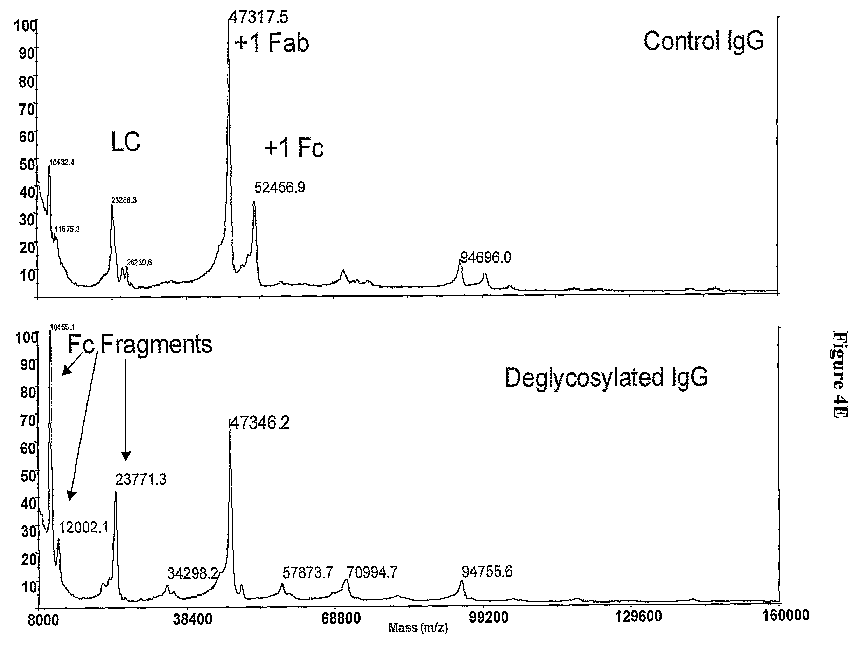

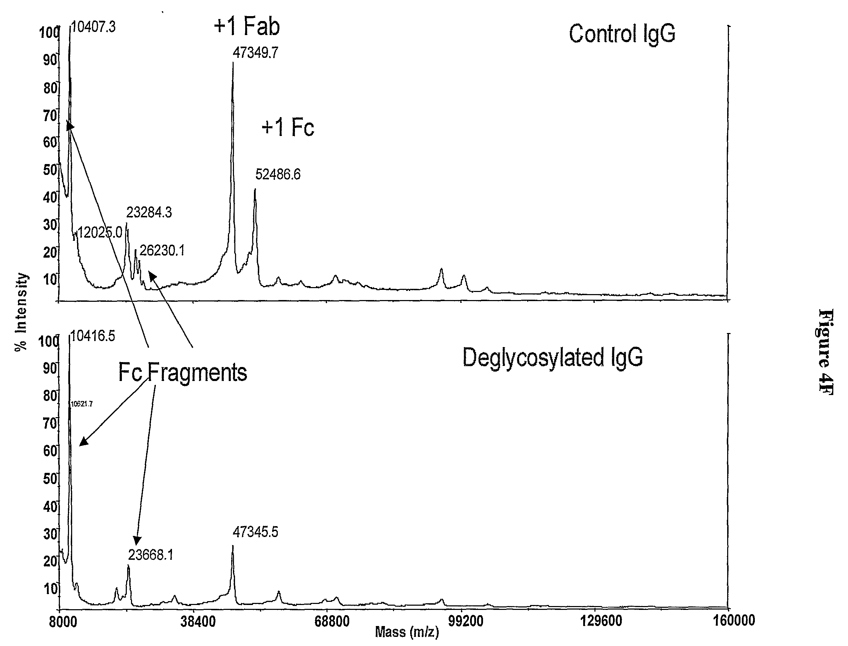

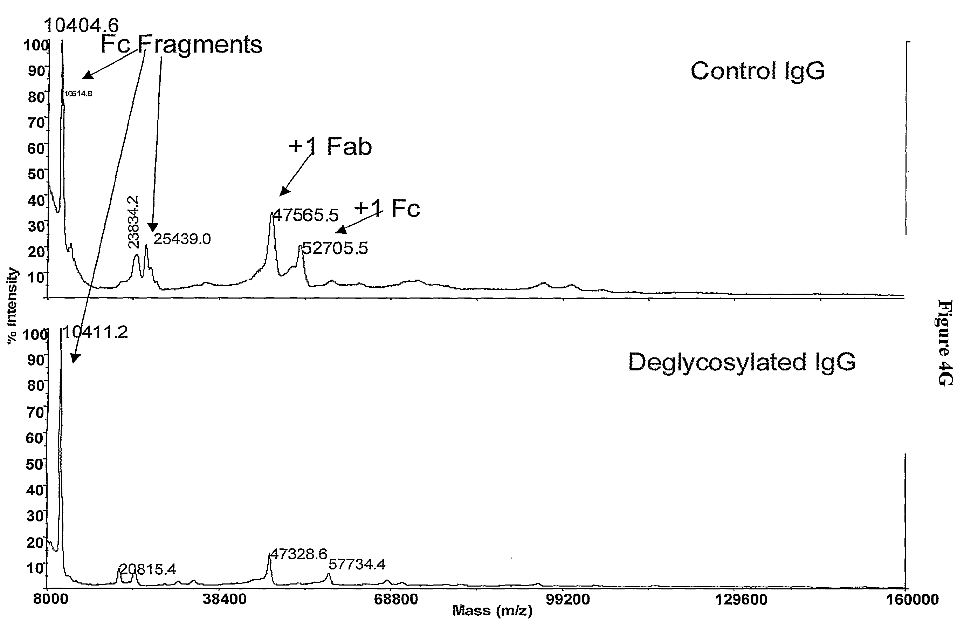

MALDI-TOF-MS data indicates that the deglycosylation of IgG under native conditions with PNGase F is complete and non-destructive (see Figs. 4A-G).

MALDI-TOF-MS analysis of aliquots withdrawn at fixed time intervals suggest that most of the deglycosylated IgG was digested by papain within one hour whereas complete digestion of control IgG (glycosylated) takes more than 4 hours. The Fc fragments (at 10 KDa and 23.7 KDa) of deglycosylated IgG are observed at 30 minutes into digestion with papain and most of the deglycosylated Fc was digested into fragments within 4 hours. Fc fragments of glycosylated IgG are observed after only 4 hours of digestion with papain at 1:50 (w/w) enzyme to substrate ratio and requires more than 24 hours to completely convert glycosylated Fc into smaller fragments at 10 KDa and 23.7 KDa.

During attempts to isolate Fc fragments from different IgG antibodies, it was observed that prior removal of CH2 domain glycans increased the rate of papain-mediated degradation of the Fc domain, making it more difficult to obtain intact Fc from deglycosylated (or aglycosylated) IgGs. Subsequent time course experiments comparing glycosylated and deglycosylated versions of both IgG antibodies and purified Fc domains showed that, in both cases, the rate of degradation of Fc in the deglycosylated molecules was at least 4-8 times faster compared to their glycosylated counterparts. These results indicate that the presence of CH2 domain glycans increases resistance to papain-mediated degradation of Fc domains. It also suggests that, given how IgGs that lack glycosylation do not seem to have a defined Fc structure and do not bind Fc receptors, papain sensitivity may constitute an additional means of assessing proper Fc structure.

### EXAMPLE 2: PAPAIN DIGESTION OF HOMOGENEOUS GLYCOFORMS

To assess the parameters of papain digestion of antibody preparations substantially homogenous with respect to their glycosylation patterns, antibody samples are enzymatically modified to produce such preparation for testing as described below.

To galactosylate purified antibody samples via enzymatic method, bovine β-1,4-galactosyltransferase (β1,4GT) and UDP-Gal obtained from Sigma Chemical Co. (St. Louis, MO) are added to the antibody samples. Recombinant rat liver α-2,3-sialyltransferase (α2,3ST), recombinant α-1,3-galactosyltransferase (α1,3GT) and CMP-Sia were obtained from Calbiochem (San Diego, CA). PNGase F was obtained from New England Biolabs (Beverly, MA) or from Prozyme (San Leandro, CA) or from Selectin BioSciences (Pleasant Hill, CA). β-Galactosidase and β-glucosaminidase from *Diplococcus pneumoniae* were obtained from either ProZyme or from Selectin BioSciences. β-Galactosidase from bovine kidney and all other enzymes were either from ProZyme or from Selectin BioSciences. NAP-5 and HiTrap protein A columns were from Pharmacia Biotech (Piscataway, NJ). All other reagents were of analytical grade.

Test antibodies for these analyses include monoclonal IgG Abs with human IgG1 and kappa constant regions expressed in transfected Sp2/0 mouse myeloma cells. The Mabs are fully human or a mouse/human chimeric MAb specific for, e.g., human TNF.

Fully galactosylated but not sialylated biantennary structures are designated G2 glycoforms. G2 antibodies were prepared by subjecting IgG samples in 100 mM MES buffer (pH 7.0) (∼10 mg in 1.0 mL of buffer) to 50 milliunits of β1,4GT, 5 µmol of UDP-Gal, and 5 µmol of MnCl2 at 37°C for 24 hours. Another aliquot of enzyme and UDP-Gal was added and the mixture was incubated for an additional 24 hours at 37°C. The regalactosylated IgG samples were purified using a HiTrap protein A column. The oligosaccharides were released by PNGase F and characterized by MALDI-TOF-MS and by HPLC as described below. The resulting preparations of Mabs were found to contain 0% sialic acid.

Fully sialylated and galactosylated antibodies are designated G2S2. The G2S2 glycoform was made by bringing IgG samples into 100 mM MES buffer, pH 7.0, (or 10 mg in 1.0 mL of buffer) using NAP-5 columns according to the manufacturer's suggested protocol. To this solution were added 50 milliunits each of β1,4GT and α2,3ST and 5 µmol each of UDP-Gal, CMP-Sia (NANA isomer), and MnCl₂. The mixture was incubated at 37°C. After 24 hours, another aliquot of enzymes was added along with the nucleotide sugars and the mixture incubated for an additional 24 hours at 37°C. The G2S2 glycoform of IgG samples was purified as described above. Using this method, sialation of an antibody preparation reached 90 to 98% G2S2 glycoform.

Test Abs were structurally analyzed by different methods. To perform MALDI-TOF-MS analysis of intact IgG Abs, IgG samples were brought into 10 mM Tris-HCl buffer, pH 7.0 and adjusted concentration to ∼1 mg/mL buffer. About 2 µl of IgG solution was mixed with 2 µl of matrix solution (the matrix solution was prepared by dissolving 10 mg sinnapinic acid in 1.0 ml of 50% acetonitrile in water containing 0.1 % trifluoroacetic acid) and 2 ml of this solution was loaded onto the target and allowed to air dry. MALDI-TOF-MS was acquired using a Voyager DE instrument from Applied BioSystems (Foster City, CA).

To perform MALDI-TOF-MS analysis of released Fc glycans, IgG samples (∼50 µg), before and after *in vitro* glycosylation reactions, were digested with PNGase F in 10 mM Tris-HCl buffer (50 µl) pH 7.0 for 4 hours at 37°C. The digestion was stopped by acidifying the reaction mixture with 50% acetic acid (∼ 5 µl) and then passing it through a cation-exchange resin column as described previously (Papac et al., 1996; Papac et al., 1998; Raju et al., 2000). These samples containing a mixture of acidic and neutral oligosaccharides were analyzed by MALDI-TOF-MS in the positive and negative ion modes, as described elsewhere (Papac et al., 1996; Papac et al., 1998; Raju et al., 2000) using a Voyager DE instrument from Applied BioSystems (Foster City, CA).

HPLC analysis of Fc glycans was done by digesting IgG samples (50 µg) in 10 mM Tris-HCl buffer (50 µl) pH 7.0 with PNGase F at 37°C for 4-8 hours. Derivatization of the released oligosaccharides with anthranilic acid (2-aminobenzoic acid) was carried out as described (Anumula KR. Anal Biochem. 2000 Jul 15;283(1):17-26). Briefly, a solution of 4% sodium acetate3H₂O (w/v) and 2% boric acid (w/v) in methanol was prepared first. The derivatization reagent was then freshly prepared by dissolving ∼30 mg of anthranilic acid (Aldrich) and ∼20 mg of sodium cyanoborohydride (Aldrich) in 1.0 ml of methanol-sodium acetate-borate solution. IgG-derived oligosaccharides (<3 nmol in 20-50 µl of water) were mixed with 0.1 ml of the anthranilic acid (AA) reagent solution in 1.6 ml polypropylene screw cap freeze vials with'O" rings (Sigma) and capped tightly. The vials were heated at 80°C in an oven or heating block (Reacti-Therm, Pierce) for 1-2 hours. After cooling the vials to room temperature, the samples were diluted with water to bring the volume to ∼0.5 ml. Derivatized oligosaccharides were purified by using NAP-5 columns.

Using a preparation that is at least 90% in the G2 or G2S2 glycoform, a papain digestion experiment as described in Example 1 is performed and analyzed to demonstrate the effect of sialic acid content on the cleavage rate and specificity of papain. Additional cleavage analyses are performed with other proteolytic enzymes using the samples as prepared in this example.

### EXAMPLE 3: PRODUCTION OF ANTIBODY FRAGMENTS USING MATRIX METALLOPROTEINASE-3

Metalloproteinases (MMPs) were purified from the supernatant of cell clones expressing recombinant human MMPs. The enzyme was activated with either 1 mM 4-aminophenylmercuric acetate (APMA; Sigma) for 1 hour at 37°C or by treating with chymotrypsin. The activated enzyme was stored at -70°C. Immunoglobulin preparations (0.5-1.0 mg/ml) were incubated with digest buffer (250 mM Tris-HCl, pH 7.4, containing 1.5 M NaCl, 50 mM CaCl₂ containing 15-60 µg/ml activated MMP) for 0-24 hours at 37°C. Aliquots were withdrawn at 0, 15, 30, 45, 60, and 120 minutes followed by 3, 4, 5, 6, 8, 12, and 24 hours. The aliquots were (about 2 microliters) with matrix solution (about 2 microliters) and 2 microliters of this mixture was loaded onto the MALDI-TOF-MS target plate and then analyzed by MALDI-TOF-MS using a Voyager DE spectrometer.

### EXAMPLE 4: PROTEOLYTIC CLEAVAGE OF PURIFIED FC

Papain was obtained from Sigma and PNGase F (peptide N-glycosidase F) was obtained from New England Biolabs. Sinapic acid was obtained from Fluka. MALDI-TOF-MS analyses were carried out using Voyager DE Biospectrometry workstation (Applied BioSystems, Foster City, CA).

Antibody (IgG) samples were deglycosylated by treating with PNGase F in 20 mM Tris-HCl buffer, pH 7.0. The deglycosylated antibody samples were purified on a protein A column (HiTrap Protein A cartridges were obtained from Amersham Biosciences) and analyzed by MALDI-TOF-MS for purity. Fc fragments of IgG were isolated and purified as described elsewhere.

Fc fragment samples (about 1 mg/ml, before and after deglycosylation) were treated with papain (1:50, w/w) in 20 mM Tris-HCl buffer, pH 7.0, containing 2 mM L-cysteine and aliquots were withdrawn at fixed time intervals (0, 15, 30, 60, 90 minutes followed by at 2, 3, 4, 5, 6, 8 and 24 hrs). The aliquots (about 2 µl) were immediately mixed with 2 µl of matrix solution (the matrix solution was prepared by dissolving 10 mg sinapic acid in 1.0 ml of 50% acetonitrile in water containing 0.1% trifluoroacetic acid) and 2 µl of this solution was loaded onto the MALDI target plate and allowed to air dry prior to the analysis.

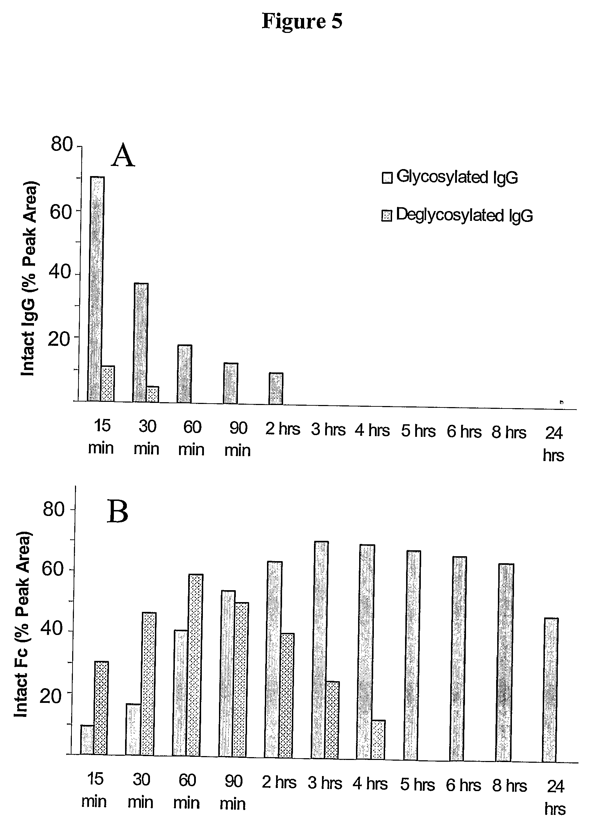

The MALDI-TOF-MS data of glycosylated and deglycosylated IgG digests was analyzed to obtain the relative % peak area data of intact IgGs and Fc fragments at all of the time points (Fig. 5). For the IgG samples, the time-course experiments indicated that within 15 minutes of digestion, more than 70% of the deglycosylated IgG was converted into Fab, Fc, and smaller Fc fragments (at m/z 10.5 KDa and 12 KDa fragments) whereas less than 50% of the glycosylated IgG was converted into fragments. After 60 minutes of incubation, no deglycosylated IgG was detectable, whereas approximately 80% of glycosylated IgG was still intact according to MALDI-TOF-MS analysis. For the Fc fragments, after 4 hrs of digestion, more than 95% of the deglycosylated Fc fragment was converted into the smaller fragments of 10.5 and 12 KDa, whereas no more than 10% of the glycosylated Fc converted into these smaller fragments at that time point. In fact, nearly 50 % of the glycosylated Fc remained undigested even after 24 hrs.

These data indicate that the glycosylated IgG is significantly more resistant to papain digestion than the deglycosylated IgG, and that the glycosylated Fc is much more resistant than deglycosylated Fc. The time-course experiment also revealed that the amount of Fab fragments from the deglycosylated and glycosylated IgGs were equivalent, suggesting that only the Fc fragments undergo overdigestion and conversion into the 10.5 KDa and 12 KDa fragments.

### EXAMPLE 5: PREPARATION OF MABS WITH SPECIFIC GLYCOFORMS

To better understand the role of oligosaccharides in increasing antibody resistance to papain, IgG preparations with homogeneous G0, G2 and G2S2 oligosaccharides were prepared using *in vitro* methods.

Preparation of G0 Glycoform To prepare homogeneous G0 glycoform, IgG samples were first treated with sialidase A to remove minor amounts of terminal sialic acid residues followed by treating with β-galactosidase to remove terminal β-galactose residues. IgG samples (10 mg in 1.0 mL) in 100 mM MES buffer (pH 7.0) were treated with 100 milliunits each of sialidase A (*A. ureafaciens*) and β-galactosidase (*D. pneumoniae*) for 24 hours at 37°C. After 24 hours, another aliquot of enzymes was added and incubated for an additional 24 hours at 37°C.

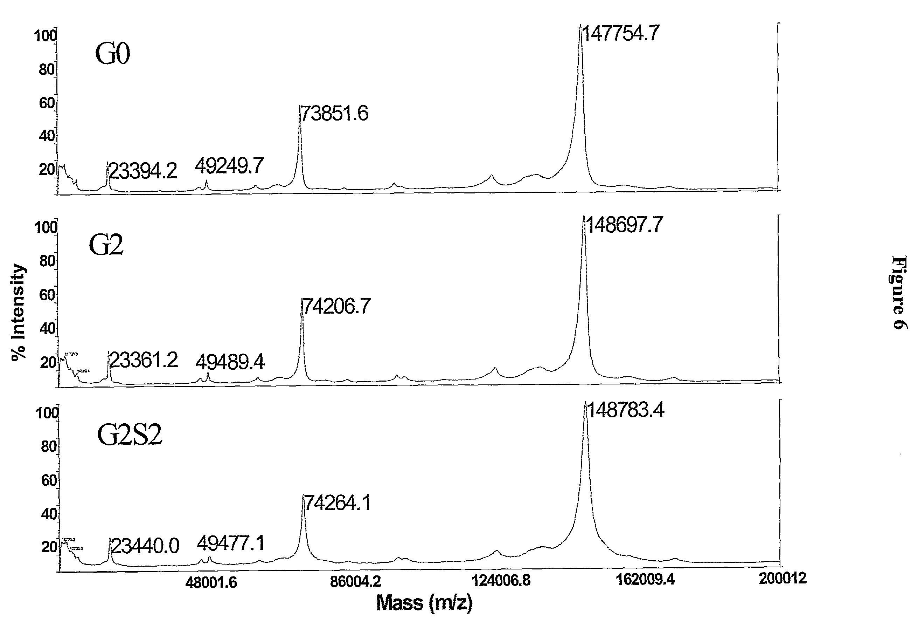

After purification on a protein A column, the resulting G0 glycoform was characterized by MALDI-TOF-MS for intact mass (Fig. 6A). The mass spectrum contained a singly charged molecular ion at m/z 147.7 KDa, a doubly charged molecular ion at m/z 73.9 KDa and a triply charged molecular ion at m/z 49.3 KDa. The mass spectrum also contained an ion at 23.4 KDa representing the free light chain produced during the laser desorption ionization. This mass spectral data indicated that the antibody was intact after treatment with enzymes to modify the Fc glycans into homogeneous G0 oligosaccharide.

The modified oligosaccharide chain, released by treating the IgG samples with PNGase F, was analyzed by MALDI-TOF-MS in the positive mode using sDHB as matrix (after purification using a cation-exchange column) and also by HPLC (after derivatizing with anthranilic acid using a reductive amination procedure as described by Anumula (1998 *supra*)*.* The MALDI-TOF-MS analysis showed a molecular ion at m/z 1486.8 that corresponds to the molecular weight of sodiated core fucosylated complex biantennary oligosaccharide terminated with GlcNAc residues. The normal phase HPLC analysis of AA-derivatized oligosaccharide afforded a single peak eluting at 20.5 min and corresponds to the elution time of AA-labeled standard core fucosylated complex biantennary oligosaccharide terminated with GlcNAc residues (data not shown) indicating that the G0 IgG glycoform sample contained more than 99% G0 oligosaccharide.

Preparation of G2 Glycoform The IgG samples were first treated with sialidase A and purified as described above. The sialidase A treated IgG samples (10 mg in 1.0 mL) in 100 mM MES buffer (pH 7.0)) were treated with 50 milliunits of β1,4GT, 5 µmol of UDP-Gal, and 5 µmol of MnCl2 at 37 °C for 24 hours. Another aliquot of enzyme and UDP-Gal was added and the mixture was incubated for an additional 24 hours at 37 °C.

After purification on a protein A column, the antibody sample was analyzed by MALDI-TOF-MS for intact mass. The mass spectrum showed a singly charged molecular ion at m/z 148.7 KDa, a doubly charged molecular ion at m/z 74.2 KDa and a triply charged molecular ion at 49.5 KDa. The mass spectrum also contained a singly charged molecular ion at m/z 23.4 KDa due to free light chain produced during laser desorption ionization. The G2 glycoform was then subjected to PNGase F treatment to release the N-linked oligsosaccharides and the released oligosaccharides were analyzed by MALDI-TOF-MS in the positive mode using sDHB as matrix. The mass spectrum showed a molecular ion at m/z 1812.1 (Fig. 6B) and this molecular ion at m/z 1812.1 corresponds to the molecular weight of sodiated core fucosylated complex biantennary oligosaccharide terminated with galactose residues. The normal phase HPLC analysis of the PNGase F released oligosaccharides after derivatization with AA exhibited a single peak and the elution time of this peak corresponds to the elution time of standard G2 oligosaccharide indicating that the G2 glycoform preparation contained intact IgG molecule with more than 99% G2 oligosaccharide.

Preparation of G2S2 Glycoform For the preparation of G2S2 glycoform, antibody samples were treated with a mixture of β**-**galactosyltransferase and α2,3-sialyltransferase in the presence of UDP-Gal, CMP-NANA and MnCl₂ in a single step. IgG samples were brought into 100 mM MES buffer (pH 7.0) (10 mg in 1.0 mL) using NAP-5 columns according to the manufacturer's suggested protocol. To this solution, were added 50 milliunits each of *β*1,4GT and α2,3ST and 5 µmol each of UDP-Gal, CMP-Sia, and MnCl₂. The mixture was incubated at 37 °C. After 24 hours, another aliquot of enzymes was added along with the nucleotide sugars and the mixture incubated for an additional 24 hours at 37 °C.

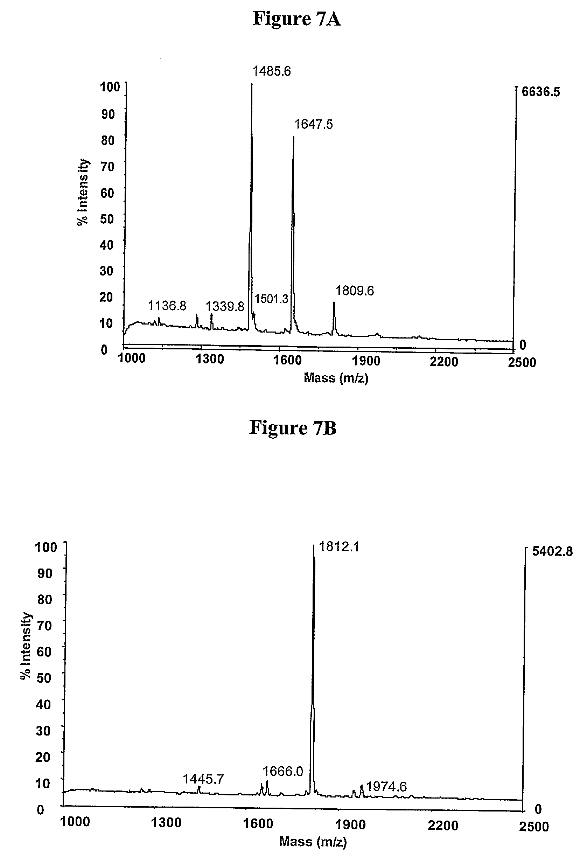

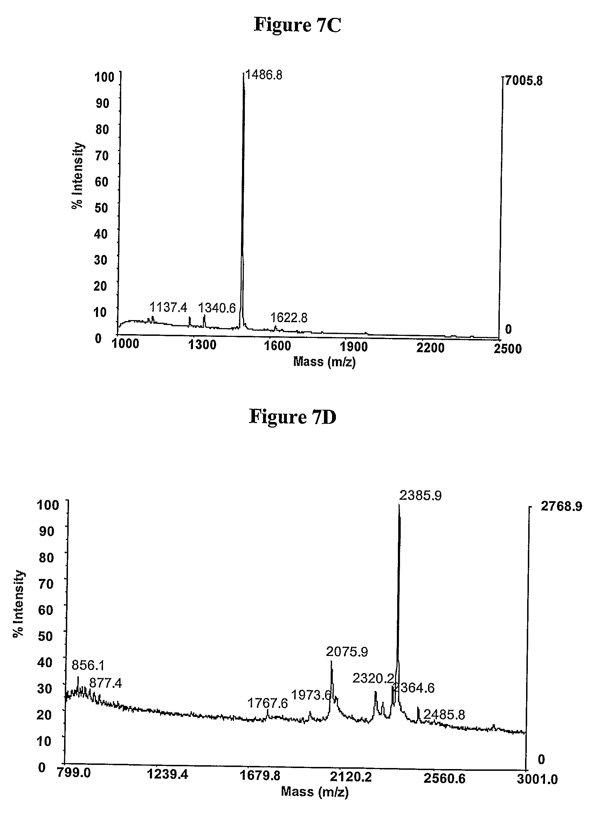

MALDI-TOF-MS analysis of the enzyme treated sample, after purification on a protein A column, showed a singly charged molecular ion at m/z ∼148.8 KDa, a doubly charged molecular ion at m/z ∼ 74.3 KDa and a triply charged molecular ion at m/z ∼49.5 Kda (Fig. 6C) suggesting that the antibody was intact and the enzyme treatment did not alter the primary structure of the antibody. The negative mode MALDI-TOF-MS and HPLC analysis of the PNGase F released oligosaccharide (Figs. 7A-D) showed that the antibody contained greater than 85% G2S2 glycoform along with minor amounts of G2S1 structure (monosialylated structures). Further, 99% of the Fc glycans contained at least one sialic acid residue. Fig. 7A shows the control (Voyager Spec #1=>NF0.7=>NR(2.00)[BP = 1485.0, 6636]), Fig. 7B shows the G2 glycoform (Voyager Spec #1=>NF0.7=>NR(2.00)[BP = 1811.4, 5403]), Fig. 7C shows the G0 glycoform (Voyager Spec #1=>NF0.7=>NF0.7=>NR(2.00)[BP = 1486.5, 7006]), and Fig. 7D shows the G2S2 glycoform (-ve mode) (Voyager Spec #1=>NF0.7[BP = 2385.4,2769]).

Size-Exclusion Chromatography To assess the amounts of aggregates present in antibody samples before and after *in vitro* modification of the glycan structures, Ab samples were analyzed by size-exclusion chromatography using an Agilent 1100 HPLC system (Agilent). A TOSO HAAS TSK 3000SWXL (Tosoh Biosep LLC) 7.8 mm × 30 cm, 5 µm column was used at ambient temperature. The mobile phase was phosphate buffered saline (PBS), and the flow rate was 0.5 ml/min. The modified IgG glycoform preparations exhibited similar chromatographic profiles to the control antibody profile indicating that the modification procedures did not create any additional aggregation and/or change in the primary structure of the protein.

### EXAMPLE 6: PAPAIN DIGESTION OF MABS WITH SPECIFIC GLYCOFORMS

To assess the relative resistance of G0, G2 and G2S2 glycoforms to papain cleavage, the IgG glycoforms and a control sample were treated with papain in the presence of cysteine at 37°C for a period of 24 hours and the digests analyzed by MALDI-TOF-MS.

### Time-Course analysis of papain digests

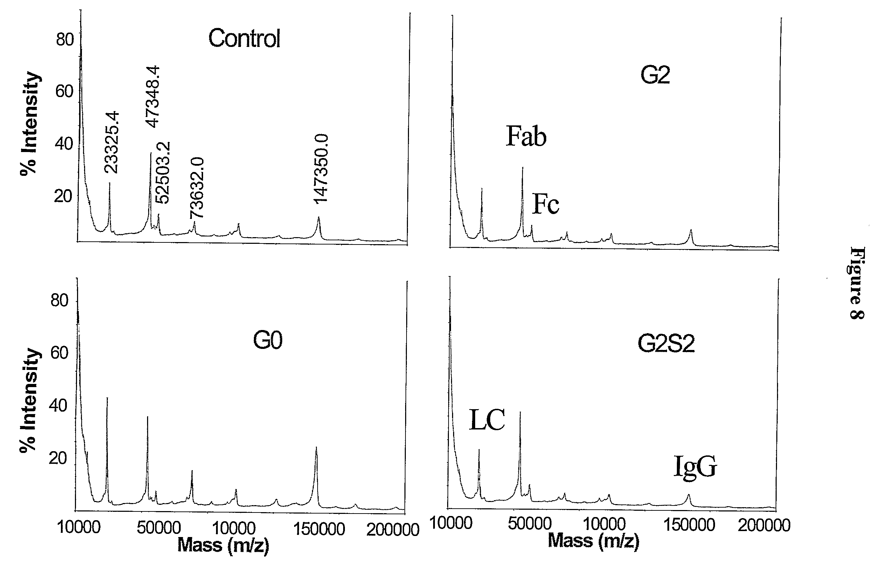

The G0, G2 and G2S2 glycoform along with the control IgG samples were treated with papain at 1:50, enzyme to substrate ratio at 37°C. From these reactions, aliquots at 0, 15, 30, 60 and 90 minutes, and at 2, 3, 4, 5, 6, 8, and 24 hours were examined by MALDI-TOF-MS. All three IgG glycoforms along with the control antibody sample were cleaved into Fab and Fc fragments, as evidenced by the presence of molecular ions at m/z ∼47.3 and ∼52.5 KDa for Fab and Fc fragments, respectively (Fig. 8).

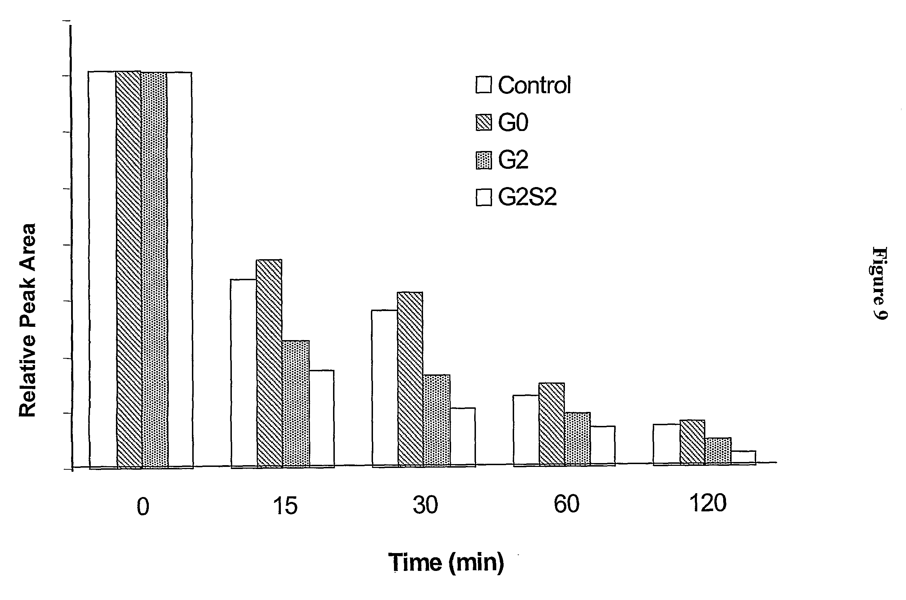

A comparison of peak height of intact IgG observed at m/z ∼ 147.5 KDa is shown in Fig. 9. Intact IgG peak at m/z ∼147.5 KDa was measured from 0 to 120 minutes, however, after 2 hours, very little intact IgG peak was observed. At 0 minute, the peak height of all of the IgG glycoforms along with the control IgG was observed to be the same. At 15 minutes, about 50% of G0, 45% of control and 35% of G2 glycoform remained undigested. In contrast, only about 25% of G2S2 glycoform remained undigested. At 30 minutes, about 45% of G0 glycoform, 40% of control antibody and ∼20% of G2 glycoform remained undigested, whereas only about 10% of G2S2 glycoform remained undigested. Therefore, the data presented in Fig. 9 suggest that the G0 glycoform is more resistant to the cleavage by papain in the CH1 domain, that produces Fab and Fc fragments as the primary products, than the other glycoforms.

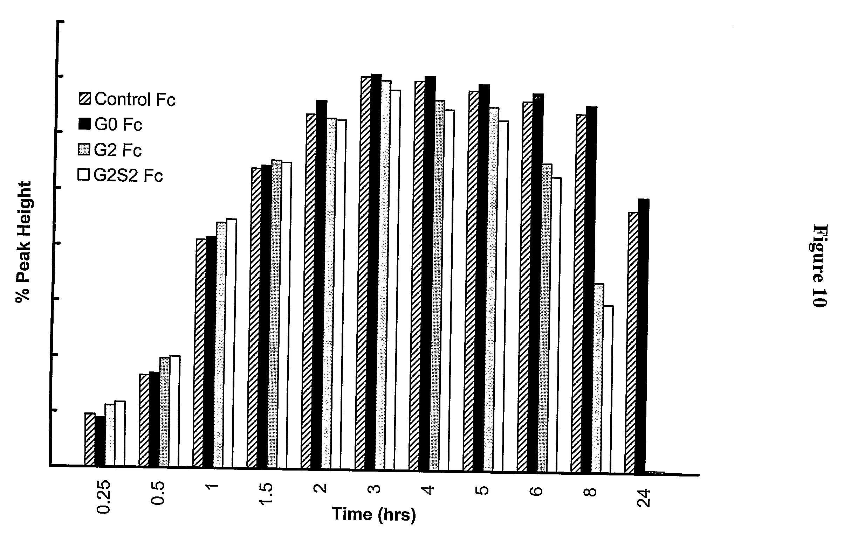

In addition to the primary papain cleavage site in the CH1 domain, IgGs can also undergo secondary cleavage in the CH2 domain of the Fc by reduced papain. To examine the resistance of IgG glycoforms to papain digestion at the secondary cleavage site, the peak heights of Fc fragments observed at m/z ∼ 52.5 KDa were compared. The peak height data of Fc fragments of G0, G2, G2S2 and control IgG is shown in Fig. 10. The relative peak height of Fc fragments of G2 and G2S2 from 0.25 hours (15 minutes) to 1 hour was about 5% more than the relative peak height of G0 glycoform; the peak heights of the Fc fragments of G0 glycoform and control IgG were almost similar. Thus, both intact IgG as G2 and G2S2 glycoforms and the Fc product itself comprising these glycoforms are more sensitive to digestion by papain. The data shown in Fig. 10 therefore exemplifies the competing rates of formation and degradation of the Fc product: at 1.5 hours time point, the peak heights of the Fc fragments of all of the glycoforms and the control IgG were almost the same. After 1.5 hours time point, the peak heights of the Fc fragments of the G2 and G2S2 glycoforms were gradually less than the peak height of the Fc fragments of the G0 glycoform and control IgG. At 6 hours time point, the peak height of the G2S2 glycoform was about 30% less than the peak height of the G0 glycoform; the G2 glycoform peak height was about 25% less than the peak height of the G0 glycoform. At 8 hours time point, the peak height of the Fc fragment of the G2S2 glycoform was about 60% less than the Fc fragment peak height of the G0 glycoform; the peak height of the Fc fragment of the G2 glycoform was about 50% less than the Fc fragment peak height of the G0 glycoform. After 0.5 hours digestion, at all the time points the Fc fragment peak height of G0 was greater than those of the G2, G2S2 and control IgG. At 24 hours time point, no appreciable Fc fragments were observed for the G2 and G2S2 glycoforms, whereas about 70% of the Fc fragments of G0 and control IgG were observed. These data indicate that the Fc fragment of the G0 glycoform is more resistant to papain digestion at the secondary cleavage site present in the CH2 domain of the Fc. Further, the data also suggest that the G2S2 glycoform is the most sensistive of the glycoforms to both primary digestion in the CH1 domain and secondary digestion in the CH2 domain.

These data suggest that the G2S2 glycoform may be more sensitive to papain digestion and the G0 glycoform may be more resistant to papain digestion than the G2 glycoform. The G2 glycoform was more resistant to papain digestion than the G2S2 glycoform, but about 50% less resistant than the G0 glycoform. These results suggested that there was differential sensitivity of IgG glycoforms to papain digestion. These differences in sensitivity seems to be both at the primary cleavage site as well as at the secondary cleavage site in the Fc.

It will be clear that the invention can be practiced otherwise than as particularly described in the foregoing description and examples. Numerous modifications and variations of the present invention are possible in light of the above teachings and, therefore, are within the scope of the appended claims.

## Claims

1. An in vitro method of enhancing the stability of an Fc-containing protein to cleavage by a protease, comprising modifying the sialylation of glycoforms in the Fc region of the Fc-containing protein by treatment with a sialidase, wherein stability to cleavage by papain is enhanced.

2. The method of claim 1, wherein the Fc-containing protein comprises an antibody in an antibody preparation.

3. The method of claim 2, wherein the altering step comprises modifying the antibody preparation so that the antibody is substantially free of sialylated glycoforms and the stability of the antibody is increased.

4. The method of claim 3, further comprising the step of modifying the antibody preparation so that the antibody is substantially homogeneous for glycoform G0.

5. The method of claim 3, wherein the modification step comprises treating the antibody preparation with sialidase A.

6. The method of claim 5, further comprising the step of treating the antibody preparation with β-galactosidase after treatment with sialidase A.

## Patentansprüche

1. In-vitro-Verfahren zur Verbesserung der Stabilität eines Fc-haltigen Proteins gegenüber Spaltung durch eine Protease, bei dem die Sialylierung von Glykoformen im Fc-Bereich des Fc-haltigen Proteins durch Behandlung mit einer Sialidase modifiziert wird, wobei die Stabilität gegenüber Spaltung durch Papain verbessert wird.

2. Verfahren nach Anspruch 1, wobei das Fc-haltige Protein einen Antikörper in einer Antikörperpräparation umfasst.

3. Verfahren nach Anspruch 2, wobei bei dem Änderungsschritt die Antikörperpräparation so modifiziert wird, dass der Antikörper weitgehend frei von sialylierten Glykoformen ist und die Stabilität des Antikörpers erhöht wird.

4. Verfahren nach Anspruch 3, bei dem in einem weiteren Schritt die Antikörperpräparation so modifiziert wird, dass der Antikörper weitgehend homogen für Glykoform G0 ist.

5. Verfahren nach Anspruch 3, wobei bei dem Modifikationsschritt die Antikörperpräparation mit Sialidase A behandelt wird.

6. Verfahren nach Anspruch 5, bei dem in einem weiteren Schritt die Antikörperpräparation nach Behandlung mit Sialidase A mit β-Galactosidase behandelt wird.

## Revendications

1. Méthode *in vitro* d'amélioration de la stabilité d'une protéine contenant Fc vis-à-vis du clivage par une protéase, comprenant la modification de la sialylation des glycoformes dans la région Fc de la protéine contenant Fc par traitement par une sialidase, où la stabilité vis-à-vis du clivage par la papaïne est améliorée.

2. Méthode selon la revendication 1, où la protéine contenant Fc comprend un anticorps dans une préparation d'anticorps.

3. Méthode selon la revendication 2, où l'état d'altération comprend la modification de la préparation d'anticorps de sorte que l'anticorps ne contienne substantiellement pas de glycoformes sialylées et que la stabilité de l'anticorps soit améliorée.

4. Méthode selon la revendication 3, comprenant en outre l'étape de modification de la préparation d'anticorps de sorte que l'anticorps soit substantiellement homogène pour la glycoforme G0.

5. Méthode selon la revendication 3, où l'étape de modification comprend le traitement de la préparation d'anticorps par la sialidase A.

6. Méthode selon la revendication 5, comprenant en outre l'étape de traitement de la préparation d'anticorps par la β-galactosidase après traitement par la sialidase A.
